# EUROPÄISCHE PATENTSCHRIFT

(11) **EP 2 361 651 B1**
(45) Veröffentlichungstag und Bekanntmachung des Hinweises auf die Patenterteilung: **27.04.2016**
(21) Anmeldenummer: 11153011.9
(22) Anmeldetag: 02.02.2011
(51) Int. Cl.: A61M 25/00, A61M 25/01, A61F 2/95, A61M 25/06, A61F 2/00

(54) **System aus einer Schutzhülse und einem medizinischen Gerät sowie Verfahren zur Herstellung desselben**
System comprising a protective sleeve and a medical device, and method for the production thereof
Système comprenant une enveloppe de protection et un appareil médical ainsi que son procédé de fabrication

(30) Priorität: 25.02.2010 US 307892 P
(43) Veröffentlichungstag der Anmeldung: 31.08.2011
(73) Patentinhaber: Biotronik AG, 8180 Bülach (CH)
(72) Erfinder: Hofmann, Andreas, 91322, Gräfenberg (DE); Filippi, Michael, 8037, Zürich (CH); Moehl, Raimund, 8127, Forch (CH); Strub, Helen, 9000, St. Gallen (CH)
(74) Vertreter: Galander, Marcus

(56) Entgegenhaltungen:
- WO-A1-01/02045
- DE-A1- 2 507 119
- US-A- 4 692 154
- US-A1- 2002 062 129
- US-A1- 2009 306 591

## Beschreibung

Die vorliegende Erfindung betrifft ein System umfassend ein medizinisches Gerät und eine Schutzhülse sowie ein Verfahren zur Herstellung und Handhabung eines derartigen Systems.

Schutzhülsen für medizinische Geräte sind gewöhnlich dazu gedacht, ein medizinisches Gerät während des Transports zu schützen. Unter einer Hülse wird ein Hohlkörper, beispielsweise ein Hohlzylinder, Hohlprisma oder Rohr verstanden, der über einen länglichen Gegenstand wie ein Rohr, einen Draht, einen Stift oder dergl. geschoben werden kann und damit zum Beispiel eine Schutz- oder Isolierfunktion ausübt. Medizinische Geräte, wie sie durch eine solche Schutzhülse geschützt werden, sind zum Beispiel Geräte für die minimalinvasive Chirurgie, insbesondere Katheter oder Implantate, beispielsweise Stents.

Eine Schutz- oder Isolierfunktion für ein medizinisches Gerät wird insbesondere für den internalen Teil eines medizinischen Geräts benötigt. Unter dem internalen Teil eines medizinischen Geräts wird dabei der Teil verstanden, der in einen menschlichen oder tierischen Körper ganz oder teilweise eingeführt wird. Der externale Teil dagegen ist der Teil eines medizinischen Geräts der nicht in den Körper eingeführt wird. Im Fall eines Katheters kann dies der Schaft sein.

Als Katheter werden allgemein Röhrchen oder Schläuche unterschiedlichen Durchmessers aus verschiedenen Materialien bezeichnet, mit denen Hohlorgane wie Harnblase, Magen, Darm, Blutgefäße oder das Herz sondiert, entleert, gefüllt oder gespült werden können. Eine spezielle Form von Kathetern stellen Ballonkatheter dar, wie sie beispielsweise in der perkutanen transluminalen koronaren Angioplastie (PTCA) angewendet werden. Bei dieser Technik wird ein verengtes Gefäß, beispielsweise ein Herzkranzgefäß, von innen durch Aufblasen eines Ballons aufgedehnt (Ballondilatation).

Heutzutage werden als Implantate für die minimalinvasive Chirurgie besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird meist mittels eines Katheters in das zu behandelnde Gefäß eingesetzt und dient dazu, das Gefäß zu stützen und/oder dauerhaft zu erweitern. Um eine Erweiterung eines Gefäßes zu erzielen, werden Stents beispielsweise auf dem Ballon eines Ballonkatheters angeordnet. Beim Aufblasen des Ballons erfolgt ein Aufweiten des Gefäßes und des Stents gleichzeitig.

Um den internalen Teil eines medizinischen Geräts in das gewünschte Hohlorgan einführen zu können, wird in der Regel ein Introducer (auch als Leitsonde oder Einführbesteck bezeichnet) in den Körper gelegt oder an dem Körper angeordnet. Der Introducer weist eine oder mehrere Zugangsöffnungen zum Einführen des oder der medizinischen Geräte auf

US 7,104,981 B2 beschreibt ein Gerät und ein Verfahren zum Einführen eines Katheters in die Aorta. Hierbei wird ein Introducer in ein Blutgefäß eingeführt und am Körper fixiert. Durch den Introducer, der proximal mit zwei Zugangsöffnungen versehen ist, können Katheter mit unterschiedlicher Form und Funktion in das Blutgefäß eingeführt werden, ohne dass bei jeder Katheterisierung ein neuer Zugang in das Blutgefäß gelegt werden muss. Der jeweilige Katheter wird direkt in eine der Zugangsöffnungen des Introducers eingeführt.

Eine Schutzhülle für einen Katheter wird in WO 01/02045 offenbart.

Medizinische Geräte wie Katheter werden in der Regel in einer Transporthülse gelagert, die steril verpackt ist. Die Transporthülse dient dabei der mechanischen Stabilisierung und dem Schutz des medizinischen Geräts. Für den bestimmungsgemäßen Einsatz herkömmlicher medizinischer Geräte muss die Transporthülse zuvor von dem Gerät entfernt werden, beispielsweise indem sie nach vorne von einem Katheter abgezogen wird. Je nach Art und Form des medizinischen Geräts ergibt sich der Nachteil, dass das medizinische Gerät nach dem Abziehen der Transporthülse nicht mehr durch diese gestützt wird und sich ggf. aufgrund seiner Flexibilität verformt, z.B. herunterhängt. Dies trifft insbesondere auf flexible Ballonkatheter zu.

Um das von der Transporthülse befreite medizinische Gerät in den Introducer einzuführen, so dass es durch diesen in ein Hohlorgan des Körpers eingeführt werden kann, muss es vom Anwender (z.B. von medizinischem Personal oder einem spezialisierten Arzt) angefasst und manuell in den Introducer eingeführt werden. Je nachdem, wie flexibel das medizinische Gerät ist, kann das Einführen in den Introducer schwierig sein. So muss beispielsweise das Einführen eines Katheters mit großer Vorsicht geschehen, um den Katheter selbst nicht zu beschädigen. Häufig fehlt aber die Zeit, beispielsweise bei medizinischen Notfällen, um mit der notwendigen Vorsicht zu arbeiten.

Ein weiterer Nachteil ergibt sich aus der Tatsache, dass medizinische Geräte, insbesondere Stents, Katheter und hierbei insbesondere der Ballon eines Katheters, häufig mit einer Beschichtung versehen sind. Beim Entfernen der Transporthülse und dem anschließenden manuellen Einführen des beschichteten Katheters in einen Introducer ist die Beschichtung verschiedenen mechanischen Beanspruchungen ausgesetzt, welche diese beschädigen oder ganz zerstören können. Neben dem Auspacken des Katheters und dem Anfassen durch den Anwender ist in Bezug auf die mechanische Beanspruchung vor allem das Einführen in den Introducer durch eine entsprechende Dichtung der Zugangsöffnung des Introducers zu nennen. Folglich kann mechanischer Schaden am Katheter verursacht oder die Beschichtung abgestreift werden.

Die Aufgabe der vorliegenden Erfindung besteht demnach darin, ein System umfassend ein medizinisches Gerät und eine Schutzhülse zu schaffen sowie ein Verfahren zur Herstellung und Handhabung eines Systems anzugeben, welche die aus dem Stand der Technik bekannten Nachteile überwinden und das medizinische Gerät und dessen Beschichtung besser schützen.

Die Aufgabe wird durch ein System nach Anspruch 1 mit einer Schutzhülse gelöst, welche in einen Introducer einführbar ist. Die Schutzhülse weist hierfür einen Grundkörper mit einem distalen Ende und einem proximalen Ende auf, auf und/oder in dessen Mantelfläche mindestens ein Anschlagelement angeordnet ist, das beim Einführen der Schutzhülse in den Introducer die Einführtiefe der Schutzhülse in den Introducer begrenzt und das vorzugsweise am distalen Ende des Grundkörpers angeordnet ist.

Mit dem distalen Ende der Schutzhülse wird das Ende beschrieben, das weiter von der Person entfernt liegt, welche die Bedienung der Schutzhülse oder des darin angeordneten medizinischen Geräts vornimmt, und beispielsweise in einen Introducer eingeführt wird. Dagegen ist das proximale Ende das dem distalen Ende der Schutzhülse gegenüber liegende Ende.

Der Vorteil des erfindungsgemäßen Systems besteht einerseits darin, dass die mechanische Beanspruchung des medizinischen Geräts verringert bzw. minimiert wird. Die erfindungsgemäße Schutzhülse muss vom Anwender zur Handhabung des medizinischen Geräts und zur Einführung in den Introducer nicht mehr entfernt werden. Das medizinische Gerät kann mit der Schutzhülse in den Introducer eingeführt werden und auch das weitere Einführen des medizinischen Geräts in den zu behandelnden Körper kann durch die Schutzhülse hindurch erfolgen. Hierdurch ist das medizinische Gerät deutlich weniger mechanischen Beanspruchungen wie Reibung oder Torsion ausgesetzt. Zudem besteht eine reduzierte Knickgefahr beim Einführen des medizinischen Geräts in den Introducer.

Ein weiterer Vorteil des erfindungsgemäßen Systems besteht darin, dass das medizinischen Gerät wie Katheter oder Stent vom Anwender bei der Handhabung nicht berührt werden muss, sondern das Anfassen im Bereich der Schutzhülse erfolgen kann, so dass eine Beschädigung und/oder Kontamination der Beschichtung, die beispielsweise aus einer pharmazeutisch aktiven Substanz bestehen kann, verhindert wird. Damit besteht auch der Vorteil, dass der direkte Kontakt des Anwenders mit der Beschichtung des medizinischen Geräts vermieden wird. Somit verhindert die beschriebene Schutzhülse nicht nur den vorzeitigen Verlust der Beschichtung, sondern schützt auch den Anwender, d.h. den Arzt oder weiteres medizinisches Personal, vor Exposition mit der Beschichtung, die eine pharmazeutisch aktive Substanz (z.B. eine zytotoxischen Substanz) enthalten kann.

Einen besonderen Vorteil bringt dabei das Anschlagelement, das verhindert, dass die Schutzhülse versehentlich zu tief in den Introducer eingeführt wird. Somit kann die Schutzhülse nicht verklemmen oder verkanten. Durch das Anschlagelement wird auch gewährleistet, dass eine an der Öffnung des Introducers angeordnete Dichtung umgangen werden kann. Beim herkömmlichen Einführen eines Katheters in einen Introducer musste demgegenüber die abdichtende Membran mit dem Katheter selbst durchdrungen werden, wodurch eine erhebliche Reibung der Membran am Katheter entsteht.

Die Schutzhülse ist insbesondere als Einführhilfe und Schutz für einen Ballonkatheter geeignet, der insbesondere auf dem Ballon eine Beschichtung, vorzugsweise mit einer pharmazeutisch aktiven Substanz, aufweisen kann. Die Schutzhülse ist insbesondere auch als Einführhilfe für einen Stent geeignet, der ebenfalls eine Beschichtung, vorzugsweise mit einer pharmazeutisch aktiven Substanz, aufweisen kann.

Das mindestens eine Anschlagelement der Schutzhülse kann in einer bevorzugten Ausführungsform als Ring oder Ringabschnitt ausgebildet sein, der auf der Mantelfläche des Grundkörpers angeordnet ist. Alternativ dazu kann das mindestens eine Anschlagelement als Rippe ausgebildet sein.

Das Anschlagelement muss den Grundkörper nicht vollständig umgeben, sondern kann auch nur auf einem Teil des Umfangs des Grundkörpers angeordnet sein. Der Ring oder Ringabschnitt besitzt gewöhnlich die größte Ausdehnung quer zur Längsrichtung, während Rippen sich beispielsweise in Längsrichtung auf oder in der Mantelfläche erstrecken können. In der Regel vergrößert das Anschlagelement den Durchmesser oder den Querschnitt der Schutzhülse in dem Abschnitt, in dem das Anschlagelement ausgebildet ist. Häufig bildet das Anschlagelement eine im Wesentlichen in radialer Richtung verlaufende Anschlagfläche aus, welche mit einer entsprechenden Anschlagfläche des Introducers wechselwirkt, um das weitere Einführen der Schutzhülse in den Introducer zu begrenzen. Die Wechselwirkung beinhaltet beispielsweise, dass die beiden Anschlagflächen aneinander anliegen.

Alternativ kann das Anschlagelement kegelstumpfförmig ausgebildet sein, wobei der kleinere Durchmesser des kegelstumpfförmigen Anschlagelements zum distalen Ende des Grundkörpers hin angeordnet ist. Beim Einführen der Schutzhülse in den Introducer wird diese so weit in den Introducer eingeführt, bis das Anschlagelement durch seine Kegelform im Innendurchmesser des Introducers anliegt und ein weiteres Einführen verhindert. Durch die kegelförmige Ausbildung des Anschlagelements ist die Begrenzung der Einführtiefe der Schutzhülse nicht vom Vorhandensein einer Anschlagfläche im Introducer abhängig.

Die Schutzhülse weist mindestens eine Sollbruchstelle auf, die sich in Längsrichtung der Schutzhülse erstreckt. Dabei kann die Sollbruchstelle als ein durchgängiger Schlitz und/oder eine durchgängige Perforation ausgeführt sein, die sich auch über die gesamte Länge der Schutzhülse erstrecken kann. Die Sollbruchstelle erleichtert das Auftrennen und Abnehmen der Schutzhülse vom medizinischen Gerät bzw. dessen Schaft. Auch hierdurch werden eine mechanische Belastung und damit eine mögliche Beschädigung des medizinischen Geräts bzw. dessen Schafts vermieden.

Der Grundkörper der Schutzhülse kann bevorzugt an seinem distalen Ende und/oder proximalen Ende einen im Wesentlichen konisch verlaufenden Abschnitt aufweisen. Der im Wesentlichen konisch verlaufende Abschnitt am proximalen Ende ermöglicht ein problemloses Aufschieben der Schutzhülse auf das medizinische Gerät, ohne das medizinische Gerät oder seine Beschichtung zu beschädigen. Der im Wesentlichen konisch verlaufende Abschnitt am distalen Ende verhindert eine Beschädigung des medizinischen Geräts oder seiner Beschichtung für den Fall, dass die Schutzhülse distal verschoben oder das medizinische Gerät nach proximal gezogen wird; auch erleichtert er das Aufschieben der Schutzhülse auf das medizinische Gerät. Ferner kann der konisch verlaufende Abschnitt am distalen Ende als Anschlagelement wirken.

Wenn an beiden Enden der Schutzhülse konisch verlaufenden Abschnitte vorgesehen sind, können diese gleich große oder verschiedene maximale Durchmesser aufweisen.

Die im Wesentlichen konisch verlaufenden Abschnitte können verschiedene Formen haben. Zum Einen können sie gerade Wände besitzen. Die Konuswinkel gegenüber der Längsachse liegen vorzugsweise zwischen 20° und 70°, besonders bevorzugt zwischen 40° und 60°. Zum Anderen können die Wände der im Wesentlichen konisch verlaufenden Abschnitte ggf. abschnittsweise nach außen oder nach innen gekrümmt sein, wobei der Krümmungsradius und die Krümmungsrichtung vom jeweiligen Anwendungszweck der Schutzhülse abhängen.

In einer Weiterbildung der Schutzhülse weist der Grundkörper insbesondere zu seinem proximalen Ende hin einen Abschnitt mit verringertem Durchmesser auf. Dieser Abschnitt mit verringertem Durchmesser (Verjüngung) führt zu einer Klemmung zwischen dem Grundkörper der Schutzhülse und dem Schaft des medizinischen Geräts. Dadurch wird bewirkt, dass sich die entstehende Reibung auf den Schaft konzentriert und die Beschichtung des medizinischen Geräts zusätzlich geschützt wird, da dessen Reibung am Grundkörper der Schutzhülse weiter verringert werden kann.

Bevorzugt ist das distale Ende des Grundkörpers der Schutzhülse mit einem Film oder einer Folie verschlossen, welcher/welche durchstoßbar oder nach dem Öffnen wieder verschließbar ausgebildet ist. Hierdurch wird das Eindringen von Fremdkörpern und/oder Kontaminationen in die Schutzhülse verhindert, welche das medizinische Gerät oder seine Beschichtung beeinträchtigen könnten. Der Film oder die Folie kann einerseits direkt vor der Handhabung der Schutzhülse, d.h. vor dem Einführen in den Introducer, vom Anwender abgezogen werden, um dem medizinischen Gerät den Weg nicht zu versperren. Andererseits kann der Film oder die Folie so dünn ausgeführt sein, dass das medizinische Gerät diesen/diese schadlos durchstoßen kann, nachdem die Schutzhülse in die Zugangsöffnung des Introducers eingeführt wurde. Auf diese Weise kann das medizinische Gerät aus der Schutzhülse heraus ohne großen Widerstand in den Introducer eingeführt werden. Der Film oder die Folie ist vorteilhaft so dünn ausgebildet, dass sie keine Schäden an einer Beschichtung mit einer pharmazeutisch aktiven Substanz auf dem medizinischen Gerät erzeugen können.

Bei einer weiteren bevorzugten Ausführungsform ist die innere Oberfläche des Grundkörpers der Schutzhülse mit einer antihaftenden und/oder Gleitfähigkeit verleihenden Beschichtung versehen, wobei die Beschichtung bevorzugt eine Verbindung aus der Gruppe umfassend PTFE (Polytetrafluorethylen) und Silicon enthält. Der Vorteil der Beschichtung ist, dass das medizinische Gerät und/oder seine Beschichtung während Lagerung und Transport nicht an der inneren Oberfläche der Schutzhülse haften können. Ferner kann beim Einführen des medizinischen Geräts in den Introducer dieses besser in ihr bzw. durch sie hindurch gleiten. Eine eventuell vorhandene Beschichtung auf dem medizinischen Gerät bleibt auf diese Weise intakt.

Aus Gründen der Optik und/oder der Hygiene und/oder der Handhabung kann die erfindungsgemäße Schutzhülse auch auf ihrer äußeren Oberfläche beschichtet sein.

Die Maße der Schutzhülse richten sich nach dem Einsatzzweck und den zu verwendenden medizinischen Geräten bzw. nach der Einführöffnung des Introducers. Vorteilhafterweise sind diese medizinischen Geräte aufgrund der Form der zu untersuchenden Hohlorgane im Wesentlichen rund oder oval ausgeführt, so dass auch die innere Oberfläche der Schutzhülse bevorzugt einen runden oder ovalen Querschnitt aufweist. Die Schutzhülse und damit der Querschnitt ihrer inneren Oberfläche können aber auch mehreckig ausgebildet sein. Insbesondere bevorzugte Größen der Schutzhülse sind Längen zwischen 50 mm und 200 mm mit Außendurchmessern des Grundkörpers von 2,2 mm bis 2,6 mm und Innendurchmessern des Grundkörpers von 1,6 mm bis 2,0 mm. Das Anschlagelement hat eine Länge (in Längsrichtung) von 5 mm bis 15 mm, bevorzugt von 8 mm bis 12 mm.

Der Grundkörper der Schutzhülse kann einen schweißbaren Kunststoff, vorzugsweise HDPE (High-Density-Polyethylen), und das Anschlagelement einen mit diesem schweißbaren Kunststoff verbindbaren Kunststoff, vorzugsweise ein Polyether-Polyamid-Blockcopolymer (z.B. Pebax^{®}), enthalten.

Das Material für den Grundkörper, bevorzugt ein Kunststoff, muss verschiedene Anforderungen erfüllen. Beispielsweise soll es vorzugsweise schweißbar sein, um ein gute Verarbeitbarkeit zu gewährleisten. Zudem muss sich das Material für eine Sterilisierung und/oder Desinfizierung mit handelsüblichen Reagenzien und/oder Verfahren eignen. Darüber hinaus muss das verwendete Material ausreichende mechanische Stabilität besitzen, um das medizinische Gerät wirkungsvoll zu schützen. Bevorzugt wird daher für den Grundkörper HDPE verwendet. Alternativ oder zusätzlich können aber auch Polyamid oder ein Polyamid-Blockcopolymer eingesetzt werden.

Das Anschlagelement wird aus einem mit dem Material des Grundkörpers verbindbaren Material hergestellt. Bevorzugt handelt es sich um ein Polyamid-Blockcopolymer, das auf den Grundkörper aufgebracht wird. Dabei kann das Element härter sein als der Grundkörper, d.h. eine höhere Shore-D-Härte aufweisen.

Alternativ kann das Anschlagelement mit dem Grundkörper integral geformt sein. Das bedeutet, dass einerseits der Grundkörper und das Anschlagelement gemeinsam aus dem gleichen Material geformt werden, oder andererseits der Grundkörper und das Anschlagelement aus unterschiedlichen Materialien hergestellt werden, wobei zum Beispiel zunächst das Anschlagelement in der Form der Schutzhülse hergestellt und anschließend mit dem Material des Grundkörpers umspritzt wird. Weiter kann die Schutzhülse mit Grundkörper und Anschlagelement gemeinsam extrudiert werden. Die Schutzhülse kann in einem weiteren Ausführungsbeispiel auch aus einem Schlauch gefertigt werden, indem ein Schlauch mit dem Außendurchmesser des Anschlagelements verwendet wird und der übrige Bereich des Grundkörpers anschließend kalibriert wird. Hierbei wird unter Kalibrieren ein Umformverfahren verstanden, bei dem die Dimensionen des Schlauchs in der Ausgangsgröße mittels Zugkräften auf die gewünschten Maße reduziert werden.

Die Schutzhülse ist aus Gründen der Hygiene und Praktikabilität als Wegwerfartikel konzipiert. Allerdings ist es auch möglich, bei entsprechender Auswahl des Werkstoffs und der Form die Schutzhülse nach Sterilisierung und/oder Desinfizierung wiederzuverwenden.

Bevorzugt ist in dem System das medizinische Gerät als Ballonkatheter ausgebildet und die Schutzhülse umgibt in diesem Fall den Ballonkatheter zumindest in dem Abschnitt, in dem der Ballon angeordnet ist.

Das erfindungsgemäße System schützt das medizinische Gerät zuverlässig vor Beschädigungen und/oder Kontaminationen. In der bevorzugten Ausführungsform, in der das medizinische Gerät ein Ballonkatheter ist, werden der Ballon, seine Faltung und ggf. die Beschichtung enthaltend mindestens eine pharmazeutisch aktive Substanz geschützt. Analog werden der Stent und/oder seine ggf. vorgesehene Beschichtung mittels einer pharmazeutisch aktiven Substanz geschützt. Im Übrigen zeigt das erfindungsgemäße System die gleichen Vorteile wie die vorstehend beschriebene Schutzhülse selbst. Darüber hinaus kann in dem erfindungsgemäßen System die Schutzhülse gleichzeitig eine Transporthülse für das zu verwendenden medizinische Gerät sein.

Wie bereits oben erläutert wurde, sind Katheter Röhrchen oder Schläuche verschiedenen Durchmessers. Ballonkatheter werden vor allem in der Angioplastie zur Erweiterung oder Wiedereröffnung eines Gefäßes eingesetzt. Ein Führungsdraht wird zuerst in das zu behandelnde Gefäß eingeschoben und anschließend wird der Ballonkatheter, der aus einem Schlauch besteht, der in einem vorgegebenen Bereich entlang des Schlauchs einen nicht dilatierten Ballon aufweist, entlang des Führungsdrahts bis zu der zu behandelnden Stelle des Gefäßes vorgeschoben, so dass der Ballon im Bereich der zu behandelnden Stelle des Gefäßes, die z.B. eine Stenose aufweist, platziert ist. Danach wird der Ballon dilatiert, d.h. entfaltet und/oder aufgedehnt, so dass die zu behandelnde Stelle wiedereröffnet oder erweitert wird. Schließlich wird der Ballon wieder entleert und entlang des Führungsdrahts wieder aus dem Gefäß entfernt. Gleichzeitig oder anschließend wird auch der Führungsdraht aus dem Gefäß zurückgezogen. Durch die Erweiterung bzw. Wiedereröffnung des Gefäßes wird der Strom der Körperflüssigkeit in dem Gefäß nicht mehr oder nicht mehr in dem zuvor vorhandenen Maße behindert. Ferner kann ein Stent in den betreffenden Gefäßabschnitt beispielsweise mithilfe des Katheters eingesetzt werden, wobei in diesem Fall der Stent zusammen mit dem Ballon dilatiert wird.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe der Taxole oder Taxane, hier vorzugsweise Paclitaxel oder Sirolimus, bestehen.

Die obige Aufgabe wird zudem von einem Verfahren zur Herstellung und Handhabung eines Systems gelöst, das ein medizinisches Gerät, vorzugsweise einen Katheter oder einen Stent, und eine Schutzhülse, welche in einen Introducer einführbar ist, insbesondere nach einer der vorstehenden Ausführungsformen, bevorzugt eines Systems nach einer der vorstehenden Ausführungsformen, umfasst. Das erfindungsgemäße Verfahren weist die folgenden Schritte auf:
a) Bereitstellen des medizinischen Geräts,
b) Aufschieben der Schutzhülse derart, dass diese das medizinische Gerät zumindest in einem Abschnitt seiner Ausdehnung in Längsrichtung umgibt, und
c) Einführen der Schutzhülse mit dem medizinischen Gerät in einen Introducer.

Vorzugsweise kann das medizinische Gerät vor dem Aufschieben der Schutzhülse in Schritt b) beschichtet werden, bevorzugt mit der mindestens einen pharmazeutisch aktiven Substanz. Auch ist es möglich, eine Beschichtung nach dem Schritt b) aufzubringen.

Bei dem erfindungsgemäßen Verfahren wird das medizinische Gerät, ggf. nach einer Beschichtung, durch die Schutzhülse geschützt. Insbesondere muss die Schutzhülse vom Anwender zur Handhabung des medizinischen Geräts nicht mehr von diesem entfernt werden. Stattdessen wird die Schutzhülse selbst teilweise in einen Introducer eingeführt. Das medizinische Gerät wird aus der Schutzhülse heraus bzw. durch die Schutzhülse hindurch in den Introducer eingeführt. Bezogen auf eine den Introducer abdichtende Membran kann somit das medizinische Gerät berührungsfrei in den Introducer eingeführt werden. Dadurch wird das medizinische Gerät keinen mechanischen Beanspruchungen wie Reibung oder Torsion ausgesetzt. Damit ergibt sich der Vorteil, dass Beschädigungen des medizinischen Geräts wie Abrieb, Schleifspuren oder dergl. sowie ein Abknicken des Geräts wirkungsvoll verhindert werden. Zudem wird vermieden, dass die Beschichtung beispielsweise mit einer pharmazeutisch aktiven Substanz abgestreift wird. Weitere Vorteile sind bereits oben im Zusammenhang mit der erfindungsgemäßen Schutzhülse erläutert.

In einer weiteren Ausführungsform des erfindungsgemäßen Verfahrens kann das System zwischen Schritt b) und c) verpackt und transportiert werden, wobei die Verpackung und der Transport gegebenenfalls nach dem Beschichten erfolgt.

In einer Weiterbildung des erfindungsgemäßen Verfahrens wird die Schutzhülse so weit in den Introducer eingeführt, bis die Einführbewegung durch ein in und/oder an der Mantelfläche des Grundkörpers der Schutzhülse angeordnetes Anschlagelement und eine Anschlagfläche des Introducers begrenzt wird. In dieser Weiterbildung verhindert das Anschlagelement durch Wechselwirkung mit der Anschlagfläche des Introducers, dass die Schutzhülse versehentlich zu tief in den Introducer eingeführt wird, so dass es sich nicht verkanten oder verklemmen kann.

Weitere Ziele, Merkmale, Vorteile und Anwendungsmöglichkeiten der Erfindung ergeben sich aus der nachfolgenden Beschreibung eines Ausführungsbeispiels anhand der Figuren.

Dabei bilden alle beschriebenen und/oder bildlich dargestellten Merkmale für sich oder in beliebiger Kombination den Gegenstand der vorliegenden Erfindung, unabhängig von ihrer Zusammenfassung in den einzelnen Ansprüchen oder deren Rückbeziehung.

Es zeigen schematisch:
- Fig. 1: eine perspektivische Ansicht eines ersten Ausführungsbeispiels der erfindungsgemäßen Schutzhülse von der Seite,
- Fig. 2: eine perspektivische Ansicht eines zweiten Ausführungsbeispiels der erfindungsgemäßen Schutzhülse von der Seite,
- Fig. 3a, b: jeweils einen Querschnitt der Zugangsöffnung eines Introducers mit der Schutzhülse gemäß Figur 1,
- Fig. 4: eine aufgeschnittene Darstellung der Schutzhülse gemäß Figur 1, in der ein Ballonkatheter angeordnet ist, und
- Fig. 5: einen Querschnitt der Schutzhülse gemäß Figur 2, in der ein Ballonkatheter angeordnet ist.

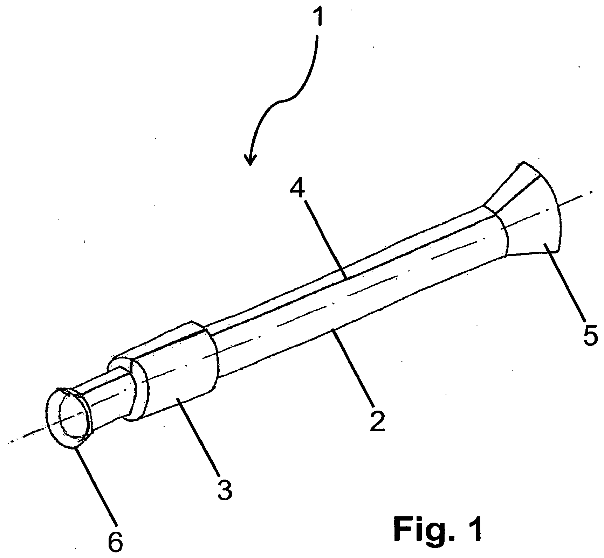

Das in Figur 1 dargestellte erste Ausführungsbeispiel zeigt eine Schutzhülse 1 für einen mit einer pharmazeutisch aktiven Substanz beschichteten Ballonkatheter 10. Die Schutzhülse 1 besteht aus einem im Wesentlichen hohlzylinderförmigen Grundkörper 2, auf dessen Mantelfläche ein Anschlagelement 3 angeordnet ist.

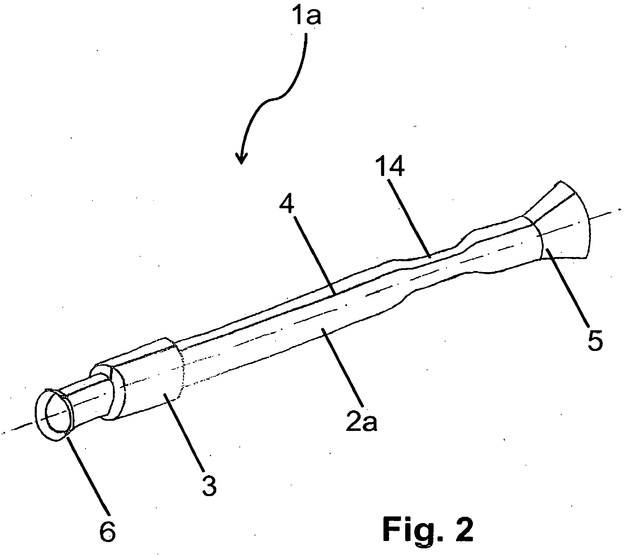

Das in Figur 2 dargestellte zweite Ausführungsbeispiel zeigt eine Schutzhülse 1a, die gegenüber dem ersten Ausführungsbeispiel zum proximalen Ende des im Wesentlichen hohlzylinderförmigen Grundkörpers 2a hin zusätzlich einen Abschnitt 14 mit verringertem Durchmesser (Verjüngung) aufweist.

Die Schutzhülsen nach beiden Ausführungsformen können eine Kathetergröße von bis zu 8 French aufnehmen.

Die Beschreibung der weiteren Elemente der erfindungsgemäßen Schutzhülse bezieht sich weitgehend sowohl auf das erste als auch auf das zweite Ausführungsbeispiel.

Das distale Ende des Anschlagelements 3 wird mit einem gewissen Abstand, beispielsweise 5 mm, vom distalen Ende der Schutzhülse 1, 1a entfernt angeordnet und umgibt den Grundkörper 2, 2a auf dessen Mantelfläche vollständig. Dabei hat es einen größeren Außendurchmesser als der Grundkörper 2, 2a in den Abschnitten, in denen weder das Anschlagelement 3 noch der erste Konus 5 oder der zweite Konus 6 angeordnet sind. Die Längsrichtung der Schutzhülse 1, 1a bzw. des Katheters 10 ist mit einer strichpunktierten Linie gekennzeichnet.

In den Figuren 1 und 2 ist das Anschlagelement 3 als Manschette oder Ring mit einer Länge von 10 mm dargestellt, die den Grundkörper 2, 2a auf seiner Mantelfläche vollständig umgibt. Alternativ kann das Anschlagelement 3 beispielweise als Ringabschnitt ausgeführt sein und/oder aus mehreren Einzelteilen bestehen, die als Rippe oder Rippen nur teilweise die Mantelfläche bedecken und sich beispielsweise in Längsrichtung erstrecken. Ebenfalls alternativ kann das Anschlagelement 3 kegelstumpfförmig ausgeführt sein derart, dass der kleinere Durchmesser zum distalen Ende des Grundkörpers 2, 2a hin angeordnet ist. Das Anschlagelement 3 ist fest mit dem Grundkörper 2, 2a verbunden oder integral mit diesem ausgebildet.

Der maximale Außendurchmesser des Anschlagelements 3 richtet sich nach dem Innendurchmesser der Zugangsöffnung 8 des Introducers 7, beträgt aber beispielsweise 1,5 mm bis 3 mm.

Der Grundkörper 2, 2a der Schutzhülse 1, 1a wird nach einem herkömmlichen Verfahren wie Spritzguss oder Extrusion hergestellt. Verwendbare Werkstoffe sind beispielsweise HDPE, PTFE, Polyamid oder Polyamid-Blockcopolymer. Das Anschlagelement 3 wird in der vorliegenden Ausführungsform bevorzugt aus einem Polyamid-Blockcopolymer hergestellt. Dieses ist gut mit dem HDPE des Grundkörpers 2, 2a durch Schweißen verbindbar.

In den dargestellten Ausführungsformen verläuft über die gesamte Länge der Schutzhülse 1, 1a in Längsrichtung auf einer Seite der Schutzhülse eine Sollbruchstelle 4 in Form eines teilweise verschlossenen Schlitzes oder einer Linie mit einer geringeren Dicke des jeweiligen Werkstoffs. Die Sollbruchstelle 4 kann aber auch entlang eines Teils der Länge der Schutzhülse 1, 1a in Längsrichtung verlaufen. Die Sollbruchstelle 4 ermöglicht es, die gebrauchte Schutzhülse 1, 1a vom Schaft des Ballonkatheters 10 abzunehmen, indem die Schutzhülse 1, 1a entlang dieser aufgetrennt wird. In den Bereichen, in denen die Mantelfläche der Schutzhülse durch einen Schlitz unterbrochen ist, liegen die Kanten des Schlitzes im Ausgangszustand so dicht aneinander an, dass ein Eindringen von Fremdkörpern und Kontaminationen wirksam verhindert wird.

Die Schutzhülse 1, 1a der dargestellten Ausführungsform besitzt am proximalen Ende des Grundkörpers 2, 2a einen nach außen weiter werdenden ersten Konus 5 (d.h. einen im Wesentlichen konisch verlaufenden Abschnitt) und am distalen Ende des Grundkörpers 2, 2a einen nach außen weiter werdenden zweiten Konus 6 (d.h. einen im Wesentlichen konisch verlaufenden Abschnitt). Dabei sind die beiden Konen 5, 6 als Trichter mit geraden Wänden dargestellt. Die Wände können jedoch auch zumindest abschnittsweise eine Krümmung aufweisen. In den dargestellten Ausführungsbeispielen ist der größte Durchmesser des ersten Konus 5 größer als der größte Durchmesser des zweiten Konus 6.

In dem ersten Ausführungsbeispiel wird die Schutzhülse 1 mit dem ersten Konus 5 auf den Ballonkatheter 10 aufgeschoben. Zudem ermöglicht der erste Konus 5 ggf. ein problemloses Aufschieben der Schutzhülse 1 auf einen nicht dargestellten Knickschutz, der optional den Ballonkatheter 10 umgeben und mit diesem zusammen in das Hohlorgan des Patienten eingeführt werden kann. Der zweite Konus 6 durchdringt beim Einführen der Schutzhülse 1 in den Introducer 7 die Membran 9 und bietet einen guten Rückhalt in der Membran 9 des Introducers 7 gegen versehentliches vorzeitiges Herausziehen. In dem zweiten Ausführungsbeispiel wird die Schutzhülse 1a von der Seite mit dem zweiten Konus 6 auf den Ballonkatheter 10 aufgeschoben.

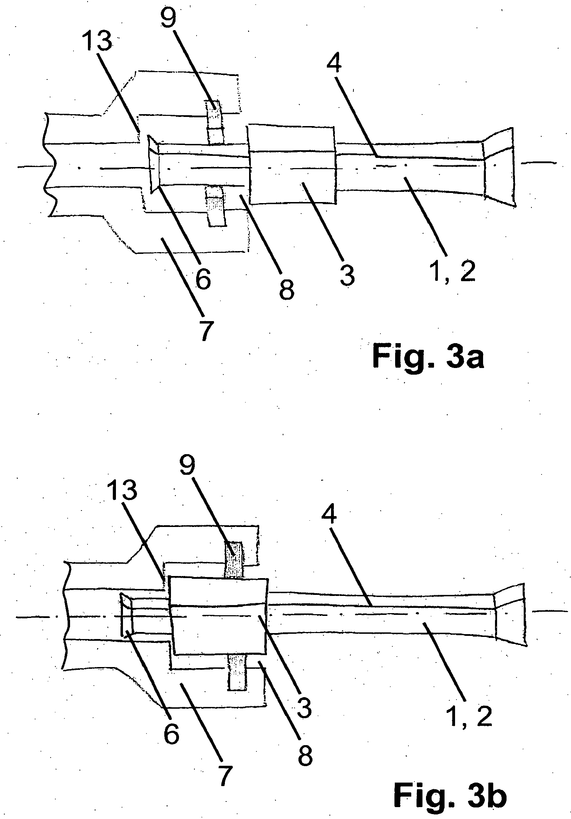

In Figur 3a ist schematisch dargestellt, wie die Schutzhülse 1 mit dem distalen Ende des Grundkörpers 2 in eine Zugangsöffnung 8 des Introducers 7 eingeführt ist. Das distale Ende wird dazu mit dem zweiten Konus 6 durch die die Zugangsöffnung 8 des Introducers 7 verschließende Membran 9 geführt. Die Membran 9, beispielsweise ein Septum, ist aus einem elastischen Material hergestellt und schließt mit dem distalen Ende des Grundkörpers 2 dicht ab, so dass keine Flüssigkeiten oder Fremdstoffe in den Introducer 7 hinein oder aus ihm heraus gelangen können. In diesem Fall bildet der zweite Konus 6 mit seinem distalen Ende ein Anschlagelement, das an einer Anschlagfläche 13 des Introducers 7 anliegt.

Figur 3b stellt eine weitere Möglichkeit der Anordnung der eingeführten Schutzhülse 1 in dem Introducer 7 dar. In dieser Konstellation wurde die Schutzhülse 1 weit in die Zugangsöffnung 8 des Introducers 7 eingeführt, so dass sie nun mit dem Anschlagelement 3 in der Zugangsöffnung 8 des Introducers 7 an der Anschlagfläche 13 anliegt. Ein tieferes Einführen der Schutzhülse 1 in den Introducer 7 wird so wirksam verhindert.

Die Darstellungen der in den Introducer 7 eingeführten Schutzhülse 1 in den Figuren 3a, 3b gelten in gleicher Weise für die Schutzhülse 1a gemäß Figur 2, auf eine gesonderte Abbildung wird daher verzichtet.

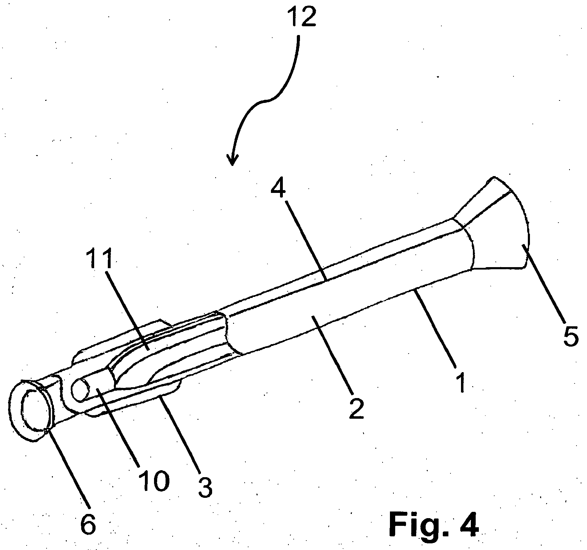

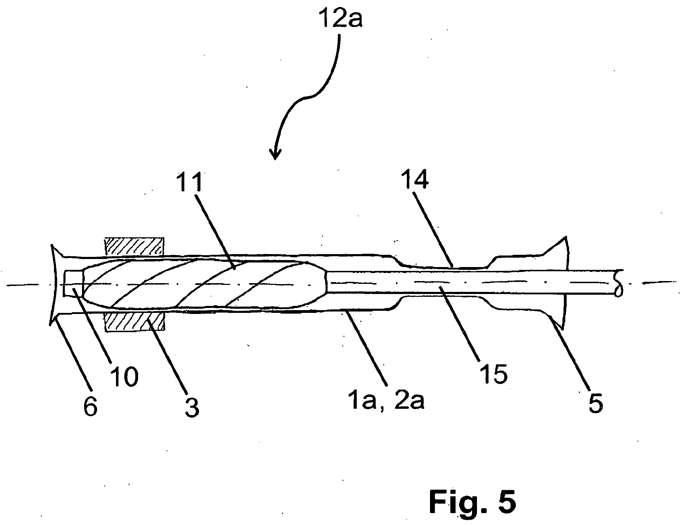

In Figur 4 wird in einer aufgeschnittenen Darstellung ein erfindungsgemäßes System 12 dargestellt, das die erfindungsgemäße Schutzhülse 1 und einen darin angeordneten Ballonkatheter 10 umfasst. Figur 5 zeigt eine Querschnittdarstellung eines weiteren erfindungsgemäßen Systems 12a, welches die erfindungsgemäße Schutzhülse 1a und ebenfalls einen darin angeordneten Ballonkatheter 10 beinhaltet.

In Figur 4 ist der Schaft des Ballonkatheters 10 nicht dargestellt. Der Ballon 11 des Ballonkatheters 10 weist vorzugsweise eine Beschichtung auf, welche eine pharmazeutisch aktive Substanz enthält. Ballonkatheter 10 werden beispielsweise für die perkutane transluminale koronare Angioplastie (PTCA) und die perkutane transluminale Angioplastie (PTA) verwendet und sind in diesem Fall häufig mit Zytostatika beschichtet.

Nach seiner Herstellung mit einem bekannten Verfahren wird der Ballonkatheter 10 zunächst von der letzten Herstellungsstation zu der Beschichtungsstation verbracht. Auf diesem Weg werden der Ballonkatheter und die Faltung des Ballons vorzugsweise mittels eines herkömmlichen Protektors geschützt, der am Ende der Herstellung montiert wird. Zur Beschichtung mit einer pharmazeutisch aktiven Substanz, beispielsweise einem Zytostatikum, wird zunächst der Protektor entfernt. Anschließend wird die Beschichtung mit einer pharmazeutisch aktiven Substanz und/oder einer anderen Verbindung nach bekannten Verfahren durchgeführt.

Nachdem die Beschichtung aufgebracht wurde, wird in dem ersten Ausführungsbeispiel die Schutzhülse 1 vorsichtig mittels des ersten Konus 5 auf den beschichteten Ballonkatheter 10 so weit aufgeschoben, bis die Schutzhülse 1 den Ballon 11 vollständig umschließt.

Im Falle des zweiten Ausführungsbeispiels wird nach dem Aufbringen der Beschichtung die Schutzhülse 1a vorsichtig mittels des zweiten Konus 6 zunächst auf den Schaft 15 des Ballonkatheters 10 und anschließend auf den beschichteten Ballon 11 selbst so weit aufgeschoben, bis die Schutzhülse 1a den Ballon 11 vollständig umschließt. Der Abschnitt 14 des Grundkörpers 2a konzentriert die anfallende Reibung im Wesentlichen auf die Klemmung zwischen dem verjüngten Abschnitt 14 und dem Schaft 15 des Ballonkatheters 10, so dass die Reibung am beschichteten Ballon 11 weiter verringert wird.

Eine Silicon-Beschichtung, die auf der inneren Oberfläche des Grundkörpers 2, 2a der Schutzhülse 1, 1a angeordnet sein kann, verhindert ein Anhaften des Ballonkatheters 10 und vermindert die Reibung weiter.

Anschließend wird das System 12, 12a aus Ballonkatheter 10 und Schutzhülse 1, 1a steril verpackt und kann problemlos transportiert werden.

Das Aufschieben der Schutzhülse 1, 1a kann natürlich auch auf unbeschichtete Ballonkatheter 10 erfolgen. Die Schutzhülse 1, 1a schützt so die dünne Wand des Ballons und dessen Faltung und verhindert ein Berühren durch den Anwender. Ggf. kann eine Beschichtung auf den Ballon 11 auch noch nach dem Aufschieben der Schutzhülse aufgebracht werden.

Zu Beginn einer medizinischen Behandlung oder einer Untersuchung wird zunächst ein Introducer 7 in ein venöses oder arterielles Gefäß beispielweise in der Leiste eingeführt und am Patienten befestigt. Der Introducer 7 wird erst am Ende der medizinischen Behandlung oder Untersuchung wieder entfernt. Der Introducer 7 dient als Führungsvorrichtung, durch dessen Inneres der Ballonkatheter 10 in das Blutgefäß eingeführt werden kann, wobei gleichzeitig die Einstichstelle abgedichtet wird.

Sobald der Introducer 7 gelegt ist, kann die Schutzhülse 1 in eine Zugangsöffnung 8 des Introducers 7 eingeführt werden. Dabei wird mit dem distalen Ende des Grundkörpers 2 der Schutzhülse 1 eine Membran 9 in der Zugangsöffnung 8 durchstoßen, die den Introducer 7 gegen die Schutzhülse 1 abdichtet. Die Schutzhülse 1 kann so weit in den Introducer 7 eingeführt werden, dass sie mit dem Anschlagelement 3 an der Anschlagfläche 13 des Introducers 7 anschlägt.

Nachdem die Schutzhülse 1 in den Introducer 7 eingeführt wurde, wird vom proximalen Ende her der Schaft des Ballonkatheters 10 vorsichtig weiter in die Schutzhülse 1 eingeschoben, wodurch der Ballonkatheter 10 mit seinem distalen Ende einen nicht dargestellten dünnen Film durchstößt, der das distale Ende des Grundkörpers 2 der Schutzhülse 1 verschließt. Alternativ kann der Film oder die Folie direkt vor dem Einführen der Schutzhülse 1 in den Introducer 7, d.h.so spät wie möglich, abgezogen werden. Bei weiterem Nachschieben des Schafts wird der Ballonkatheter 10 ganz in den Introducer 7 und weiter in das Blutgefäß eingeführt.

Wenn der Ballonkatheter 10 weit genug in das Gefäß eingeführt wurde, kann die Schutzhülse 1 von dem Katheter entfernt werden. Dazu wird sie zunächst aus der Membran 9 des Introducers 7 herausgezogen, wobei sich die Membran 9 wieder schließt und den Introducer 7 sofort wieder abdichtet. Nun hängt die Schutzhülse 1 frei auf dem Schaft. Entlang der Sollbruchstelle 4 wird die Schutzhülse 1 geöffnet und anschließend so weit aufgebogen, so dass sie ohne Beschädigung des Schafts abgenommen werden kann. Bei der weiteren Behandlung oder Untersuchung stört oder behindert die Schutzhülse 1 nun die weitere Behandlung nicht.

### Bezugszeichenliste:

- 1, 1a: Schutzhülse
- 2, 2a: Grundkörper
- 3: Anschlagelement
- 4: Sollbruchstelle
- 5: erster Konus
- 6: zweiter Konus
- 7: Introducer
- 8: Zugangsöffnung des Introducers 7
- 9: abdichtende Membran
- 10: Ballonkatheter
- 11: Ballon
- 12, 12a: System
- 13: Anschlagfläche
- 14: Abschnitt mit verringertem Durchmesser
- 15: Schaft

## Patentansprüche

1. System (12, 12a) umfassend ein medizinisches Gerät und eine Schutzhülse (1, 1a), welche in einen Introducer (7) einführbar ist, wobei die Schutzhülse (1, 1a) einen Grundkörper (2, 2a) mit einem distalen Ende und einem proximalen Ende aufweist, in und/oder auf dessen Mantelfläche mindestens ein Anschlagelement (3) angeordnet ist, das beim Einführen der Schutzhülse (1, 1a) in den Introducer (7) die Einführtiefe der Schutzhülse (1, 1a) in den Introducer (7) begrenzt, und wobei die Schutzhülse (1, 1a) das medizinische Gerät zumindest in einem Abschnitt der Ausdehnung des medizinischen Geräts in Längsrichtung umgibt,
**dadurch gekennzeichnet, dass** die Schutzhülse (1, 1a) mindestens eine Sollbruchstelle (4) aufweist, die sich in Längsrichtung der Schutzhülse (1, 1a) erstreckt

2. System (12, 12a) nach Anspruch 1, wobei das Anschlagselement (3) der Schutzhülse (1, 1a) am distalen Ende des Grundkörpers (2, 2a) angeordnet ist.

3. System (12, 12a) nach Anspruch 1 oder 2, **dadurch gekennzeichnet, dass** das Anschlagelement (3) als Ring, Ringabschnitt oder Kegelstumpf ausgebildet ist.

4. System (12, 12a) nach Anspruch 1 oder 2, **dadurch gekennzeichnet, dass** das Anschlagelement (3) als Rippe ausgebildet ist.

5. System (12, 12a) nach einem der vorhergehenden Ansprüche, **dadurch gekennzeichnet, dass** der Grundkörper (2, 2a) an seinem distalen Ende und/oder proximalen Ende einen im Wesentlichen konisch verlaufenden Abschnitt (5, 6) aufweist.

6. System (12, 12a) nach einem der vorhergehenden Ansprüche, **dadurch gekennzeichnet, dass** der Grundkörper (2a) an seinem proximalen Ende einen Abschnitt (14) mit verringertem Durchmesser aufweist.

7. System (12, 12a) nach einem der vorhergehenden Ansprüche, **dadurch gekennzeichnet, dass** das distale Ende des Grundkörpers (2, 2a) mit einem Film oder einer Folie verschlossen ist, welche durchstoßbar oder nach dem Öffnen wieder verschließbar ausgebildet ist.

8. System (12, 12a) nach einem der vorhergehenden Ansprüche, **dadurch gekennzeichnet, dass** die innere Oberfläche des Grundkörpers (2, 2a) mit einer antihaftenden und/oder Gleitfähigkeit verleihenden Beschichtung versehen ist, wobei die Beschichtung bevorzugt eine Verbindung aus der Gruppe umfassend PTFE und Silicon enthält.

9. System (12, 12a) nach einem der vorhergehenden Ansprüche, **dadurch gekennzeichnet, dass** der Grundkörper (2, 2a) einen schweißbaren Kunststoff, vorzugsweise HDPE, und das Anschlagelement (3) einen mit diesem schweißbaren Kunststoff verbindbaren Kunststoff, vorzugsweise Polyamid oder ein Polyether-Polyamid-Blockcopolymer, enthält.

10. System (12, 12a) nach einem der vorhergehenden Ansprüche umfassend ein medizinisches Gerät, welches ein Katheter (10) oder ein Stent ist.

11. System (12, 12a) nach einem der vorhergehenden Ansprüche, **dadurch gekennzeichnet, dass** das medizinische Gerät als Ballonkatheter (10) ausgebildet ist und die Schutzhülse (1, 1a) den Ballonkatheter (10) zumindest in dem Abschnitt umgibt, in dem der Ballon (11) angeordnet ist.

12. Verfahren zur Herstellung und Handhabung eines Systems (12, 12a) nach einem der vorhergehenden Ansprüche mit den folgenden Schritten
a) Bereitstellen des medizinischen Geräts (10),
b) Aufschieben der Schutzhülse (1, 1a) derart, dass diese das medizinische Gerät (10) zumindest in einem Abschnitt seiner Ausdehnung in Längsrichtung umgibt, und
c) Einführen der Schutzhülse (1, 1a) mit dem medizinischen Gerät in einen Introducer (7).

13. Verfahren nach Anspruch 12, **dadurch gekennzeichnet, dass** das System (12, 12a) nach den Ansprüchen 1 bis 11 zwischen Schritt b) und c) verpackt und transportiert wird.

14. Verfahren nach einem der Ansprüche 12 und 13, **dadurch gekennzeichnet, dass** die Schutzhülse (1, 1a) so weit in den Introducer (7) eingeführt wird, bis die Einführbewegung durch ein an der Mantelfläche des Grundkörpers (2, 2a) der Schutzhülse (1, 1a) angeordnetes Anschlagelement (3) und eine Anschlagfläche (13) des Introducers (7) begrenzt wird.

15. Verfahren nach einem der Ansprüche 12 bis 14, **dadurch gekennzeichnet, dass** das medizinische Gerät (10) vor Schritt b) beschichtet wird, bevorzugt mit mindestens einer pharmazeutisch aktiven Substanz.

## Claims

1. A system (12, 12a), comprising a medical device and a protective sleeve (1, 1a), which can be inserted into an introducer (7), wherein the protective sleeve (1, 1a) comprises a main body (2, 2a) which has a distal end and a proximal end, and in and/or on the lateral surface of which at least one stop element (3) is disposed, which during insertion of the protective sleeve (1, 1a) into the introducer (7) limits the insertion depth of the protective sleeve (1, 1a) into the introducer (7), and wherein the protective sleeve (1, 1a) surrounds the medical device at least in a portion of the extension of the medical device in the longitudinal direction,
**characterised in that** the protective sleeve (1, 1a) has at least one predetermined breaking point (4), which extends in the longitudinal direction of the protective sleeve (1, 1a).

2. The system (12, 12a) according to Claim 1, wherein the stop element (3) of the protective sleeve (1, 1a) is arranged at the distal end of the main body (2, 2a).

3. The system (12, 12a) according to Claim 1 or 2, **characterised in that** the stop element (3) is designed as a ring, ring portion or truncated cone.

4. The system (12, 12a) according to Claim 1 or 2, **characterised in that** the stop element (3) is designed as a rib.

5. The system (12, 12a) according to one of the preceding claims, **characterised in that** the main body (2, 2a) has, at its distal end and/or proximal end, a substantially conically extending portion (5, 6).

6. The system (12, 12a) according to one of the preceding claims, **characterised in that** the main body (2a) has, at its proximal end, a portion (14) of reduced diameter.

7. The system (12, 12a) according to one of the preceding claims, **characterised in that** the distal end of the main body (2, 2a) is closed by a film or a foil, which can be penetrated or resealed after opening.

8. The system (12, 12a) according to one of the preceding claims, **characterised in that** the inner surface of the main body (2, 2a) is provided with a non-stick coating and/or a coating enhancing the gliding properties, wherein the coating preferably comprises a compound selected from the group consisting of PTFE and silicone.

9. The system (12, 12a) according to one of the preceding claims, **characterised in that** the main body (2, 2a) comprises a weldable plastic material, optionally HDPE, and the stop element comprises a plastic material which can be bonded to the weldable plastic, preferably polyamide or a polyether-polyamide block copolymer.

10. The system (12, 12a) according to one of the preceding claims, comprising a medical device, which is a catheter (10) or a stent.

11. The system (12, 12a) according to one of the preceding claims, **characterised in that** the medical device is designed as a balloon catheter (10) and the protective sleeve (1, 1a) surrounds the balloon catheter (10) at least in the portion in which the balloon (11) is disposed.

12. A method for producing and handling a system (12, 12a) according to one of the preceding claims, said method comprising the following steps
a) providing the medical device (10),
b) sliding on the protective sleeve (1, 1a) such that it surrounds the medical device (10) in the longitudinal direction at least in one portion of the extension thereof, and
c) inserting the protective sleeve (1, 1a) with the medical device into an introducer (7).

13. The method according to Claim 12, **characterised in that** the system (12, 12a) according to one of Claims 1 to 11 is packaged between steps b) and c) and transported.

14. The method according to one of Claims 12 and 13, **characterised in that** the protective sleeve (1, 1a) is inserted into the introducer (7) until the insertion movement is limited by a stop element (3) disposed on the lateral surface of the main body (2, 2a) of the protective sleeve (1, 1a) and a stop surface (13) of the introducer (7).

15. The method according to one of Claims 12 to 14, **characterised in that** the medical device (10) is coated prior to step b), preferably using at least one pharmaceutically active substance.

## Revendications

1. Système (12, 12a) comprenant un appareil médical et un manchon de protection (1, 1a), lequel peut être inséré dans un dispositif d'introduction (7), où le manchon de protection (1, 1a) présente un corps de base (2, 2a), avec une extrémité distale et une extrémité proximale, au moins un élément de butée (3) est disposé dans, et/ou sur, sa surface de gainage qui, lors de l'introduction du manchon de protection (1, 1a) dans le dispositif d'introduction (7), limite la profondeur d'introduction du manchon de protection (1, 1a) dans le dispositif d'introduction (7), et où le manchon de protection (1, 1a) entoure l'appareil médical au moins sur une section de l'extension de l'appareil médical dans la direction longitudinale, **caractérisé en ce que** le manchon de protection (1, 1a) présente au moins un point prévu pour une rupture (4) qui s'étend dans la direction longitudinale du manchon de protection (1, 1a).

2. Système (12, 12a) selon la revendication 1, dans lequel l'élément de butée (3) du manchon de protection (1, 1a) est disposé à l'extrémité distale du corps de base (2, 2a).

3. Système (12, 12a) selon la revendication 1 ou 2, **caractérisé en ce que** l'élément de butée (3) est conçu sous la forme d'un anneau, d'une section d'anneau ou d'un cône tronqué.

4. Système (12, 12a) selon la revendication 1 ou 2, **caractérisé en ce que** l'élément de butée (3) est conçu sous la forme d'une nervure.

5. Système (12, 12a) selon l'une des revendications précédentes, **caractérisé en ce que** le corps de base (2, 2a) présente à son extrémité distale, et/ou proximale, une section (5, 6) s'étendant essentiellement de manière conique.

6. Système (12, 12a) selon l'une des revendications précédentes, **caractérisé en ce que** le corps de base (2a) présente une section (14) avec un diamètre réduit à son extrémité proximale.

7. Système (12, 12a) selon l'une des revendications précédentes, **caractérisé en ce que** l'extrémité distale du corps de base (2, 2a) est fermée avec un film ou une feuille, lequel(laquelle) peut être conçu(e) percé(e) ou peut de nouveau être refermé(e) après l'ouverture.

8. Système (12, 12a) selon l'une des revendications précédentes, **caractérisé en ce que** la surface intérieure du corps de base (2, 2a) est munie d'un revêtement antiadhésif et/ou est conférée des propriétés de glissement, où le revêtement contient de préférence un composé provenant du groupe comprenant le PTFE et le silicone.

9. Système (12, 12a) selon l'une des revendications précédentes, **caractérisé en ce que** le corps de base (2, 2a) contient une matière synthétique pouvant être soudée, de préférence un PE HD, et l'élément de butée (3) contient une matière synthétique pouvant s'unir avec cette matière synthétique pouvant être soudée, de préférence un polyamide ou un copolymère séquencé polyéther-polyamide.

10. Système (12, 12a) selon l'une des revendications précédentes, comprenant un appareil médical, lequel est un cathéter (10) ou un stent.

11. Système (12, 12a) selon l'une des revendications précédentes, **caractérisé en ce que** l'appareil médical est conçu sous la forme d'un cathéter à ballon (10) et que le manchon de protection (1, 1a) entoure le cathéter à ballon (10) au moins sur la section dans laquelle est disposé le ballon (11).

12. Procédé de fabrication et de maniement d'un système (12, 12a) selon l'une des revendications précédentes avec les étapes suivantes
a) mise à disposition d'un appareil médical (10),
b) poussée du manchon de protection (1, 1a) vers le haut de telle sorte qu'au moins une section de l'extension de celui-ci en direction longitudinale entoure l'appareil médical (10), et
c) introduction du manchon de protection (1, 1a) avec l'appareil médical dans un dispositif d'introduction (7).

13. Procédé selon la revendication 12, **caractérisé en ce que** le système (12, 12a) selon les revendications 1 à 11 est emballé et transporté entre les étapes b) et c).

14. Procédé selon l'une des revendications 12 et 13, **caractérisé en ce que** le manchon de protection (1, 1a) est inséré dans le dispositif d'introduction (7) aussi loin jusqu'à ce que le mouvement d'insertion se trouve limité par un élément de butée (3) disposé sur une surface de gainage du corps de base (2, 2a) du manchon de protection (1, 1a) et une surface de butée (13) du dispositif d'introduction (7).

15. Procédé selon l'une des revendications 12 à 14, **caractérisé en ce qu'**avant l'étape b), l'appareil médical (10) est revêtu, de préférence avec au moins une substance pharmaceutique active.
